(19) **Europäisches Patentamt / European Patent Office / Office européen des brevets**

(11) **EP 4 670 766 A1**

(12) # EUROPEAN PATENT APPLICATION

(43) Date of publication:
31.12.2025 Bulletin 2026/01

(21) Application number: 24185275.5

(22) Date of filing: 28.06.2024

(51) International Patent Classification (IPC):
**A61M 15/00** *(2006.01)*

(52) Cooperative Patent Classification (CPC):
(C-Sets available)
**A61M 15/0086; A61M 15/0015;** A61M 15/0016;
A61M 15/0018; A61M 15/0021; A61M 2202/0208;
A61M 2205/42                                              (Cont.)

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(71) Applicant: **Stamford Devices Limited**
**Galway, H91 HE94 (IE)**

(72) Inventors:
• **HAYES, Michael-John**
  **Galway (IE)**
• **CASEY, Micheal**
  **Galway (IE)**

(74) Representative: **Weldon O'Brien Ltd.**
**Shannon Lodge**
**Casement Road**
**Bandon**
**County Cork, P72 TN24 (IE)**

(54) **AN AEROSOL DELIVERY DEVICE**

(57) An aerosol delivery device has a housing (2) defining a hand-held chamber. There is an air inlet (50) with a one-way valve to allow inflow during inhalation. An aerosol port (6) is for delivery of aerosol into the chamber. A patient interface (3) is detachable and has a one-way valve (22) to allow exhaust outflow during exhalation. The inlet valve has an annular flap (55) with a central hub (54) through which a retainer (52) extends axially and extends radially to hold the hub (54) in place between the retainer and a valve seat (60). The retainer does not press the hub (54) against the valve seat, the gap (H-V) between the retainer and the valve seat being greater than the axial dimension of the hub (54). There is a rim (65) providing that the vale flap (55) edge is raised or pre-loaded by an extent (P) from a resting unconstrained configuration. The retainer (52) has a maximum radial dimension (D 1) which is less than a radial dimension (D2) of the hub by at least 10%. These features achieve excellent valve operation with reduced noise.

Fig.5

EP 4 670 766 A1

(52) Cooperative Patent Classification (CPC): (Cont.)

C-Sets
A61M 2202/0208, A61M 2202/0007

**Description**

Introduction

This invention relates to the delivery of aerosol to patients in response to spontaneous breathing.

EP2868339 (Stamford Devices Ltd.) describes such a delivery system, having a main body, an aerosol port, an air inlet valve in the base, an oxygen port also in the base, and a mouthpiece.

The present invention is directed towards reducing audible valve hum during inhalation whilst ensuring adequate valve sealing.

Summary of the Invention

We describe an aerosol delivery device comprising:

a housing defining a chamber,
an air inlet with a one-way valve to allow inflow during inhalation,
an aerosol port for delivery of aerosol into the chamber, and
a patient interface with a one-way valve to allow exhaust outflow during exhalation, wherein:

the inlet valve comprises an annular flap with a central hub through which a retainer extends axially, and the retainer extends radially to hold the hub in place between the retainer and a valve seat,
the retainer does not press the hub against the valve seat, a gap between the retainer and the valve seat being greater than the axial dimension of the hub.

In some preferred examples, the retainer has a maximum radial dimension D1 which is less than a radial dimension D2 of the hub.

In some preferred examples, the retainer maximum radial dimension is less than the radial dimension of the hub by at least 10%.

In some preferred examples, the retainer maximum radial dimension D1 is in the range of 80% to 90% and preferably 83% to 87% of the maximum radial dimension D2 of the hub.

In some preferred examples, the outer edge of the valve flap is raised out of its resting plane by a pre-load displacement distance by a valve rim at the outer periphery of the valve flap on a valve seat, in which:

$$P = S - H + V$$

where,

S is the height of the valve rim,
H is the axial dimension between the valve seat and the retainer, and
V is the thickness of the valve hub.

In some preferred examples, the pre-load displacement distance P is in the range of 18% and 157% of the thickness of the valve annular flap.

In some preferred examples, the pre-load displacement distance P is in the range of 48% to 128% of the thickness of the valve annular flap.

In some preferred examples, the valve pre-load of the thickness of the valve annular flap distance P is in the range of 0.5% to 3.5% of the diameter of the valve flap.

Detailed Description of the Invention

The invention will be more clearly understood from the following description of some embodiments thereof, given by way of example only with reference to the accompanying drawings in which:

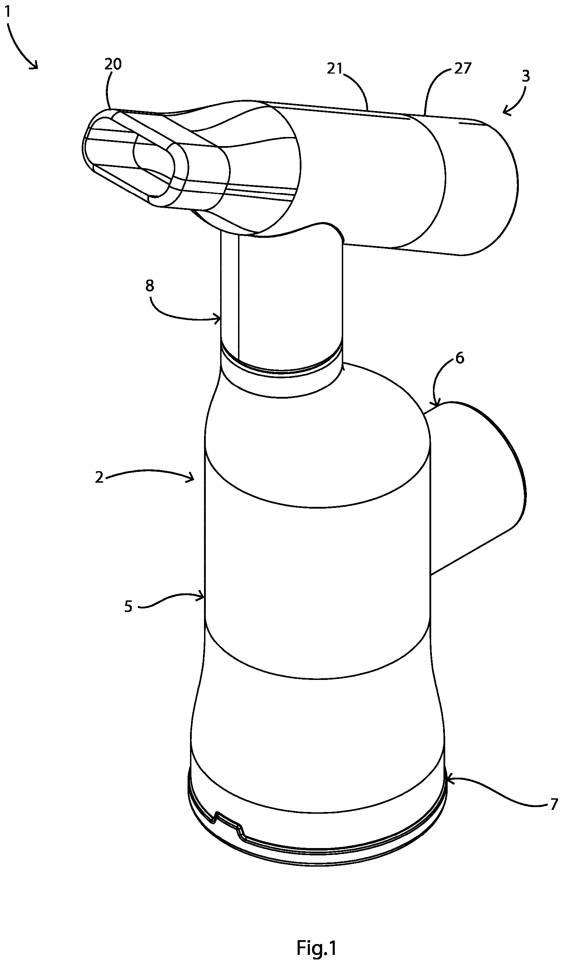

Fig. 1 is a perspective view of an aerosol delivery device of the invention;

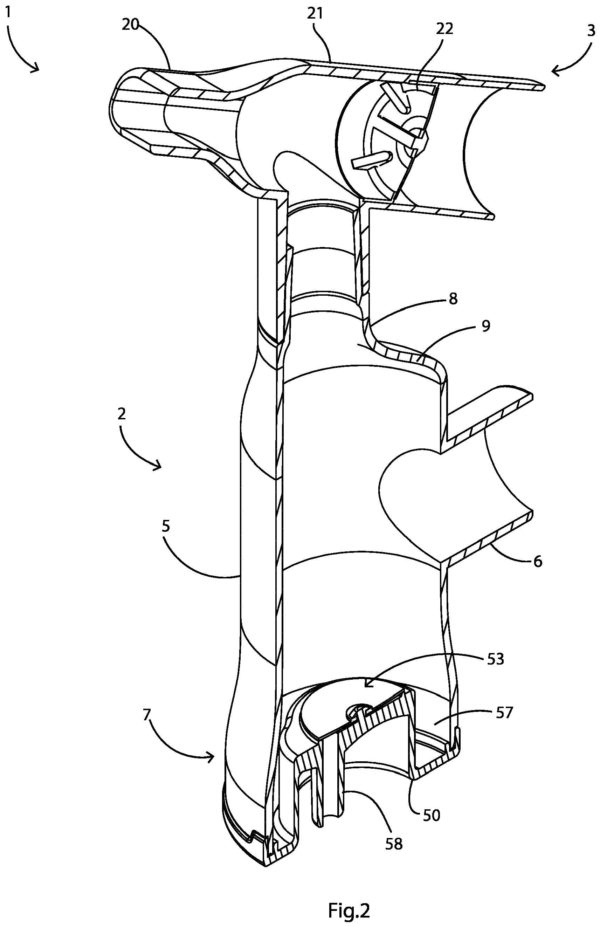

Fig. 2 is a cut-away perspective view of the device;

Figs. 3(a), (b), and (c) are a plan view, a cross-sectional view, and a side view of a valve flap of the device;

Figs. 4(a) and (b) are a plan view and a cross-sectional view of the valve, and Fig. 4(c) is an enlarged cross-sectional view showing how the valve flap is retained;

Fig. 5 is a sectional diagram showing relative dimensions and ranges of freedom of motion of the valve flap in use;

Fig. 6 is a plot of acoustic dB levels vs. time for a baseline of Active Servo Lung (ASL, simulated breathing apparatus) with only the nebulizer providing aerosol; and

Fig. 7 is a plot of acoustic dB levels vs. time for a prior art device, Fig. 8 is an equivalent plot for a device of the invention, and Fig. 9 is a plot showing differences in operating pressure of inhalation between the prior art and invention devices.

Referring to the drawings there is illustrated an aerosol delivery device 1 according to the invention which comprises a main housing 2 and a detachable mouthpiece 3.

The housing 2 defines a chamber 5 in which air and aerosol are mixed before being inhaled by a patient via the mouthpiece 3.

The housing 2 has an aerosol port 6 for connection to an outlet conduit of a nebulizer such as the Aerogen Solo™, and a base 7 with an ambient air inlet which is normally closed by an inlet valve 53 at the upper end of a boss 50.

The housing 2 also has a patient port 8 for receiving the mouthpiece 3, or in other examples a face mask. The mouthpiece 3 has a generally tubular body 21 with a mouth 20 and a valve 22 leading to an exhaust outlet 27. Exhaled air is exhausted through the mouth 20 and the valve 22 to prevent recirculation through the chamber 5 which would adversely affect dose efficiencies.

The aerosol port 6 is located in a side of the housing 2 for delivery of aerosol into the chamber 5 between the inlet valve 53 and the patient port 8, generally perpendicular to the flow of air through the chamber 5.

The inlet valve 53 and the exhaust valve 22 are one-way breath-actuated and move from an inspiration configuration in which the inlet valve 53 is open and the exhaust valve 22 is closed to an exhalation configuration in which the inlet valve 53 is closed and the exhaust valve 22 is open.

The base 7 comprises the boss 50 extending upwardly into the housing 2 volume. The boss is spaced-apart inwardly of the main body of the housing 2 to define a reception space or well 57 around the boss 50. This facilitates collection of any rain-out within the chamber 5. The inlet valve 53 has an annular flap 55 around a hub 54 and is of a flexible polymeric material such as Elastosil R401-40™ (Wacker, Munich, Germany). The flap hub 54 is held by a mounting element or retainer 52 which extends through a central on-axis aperture 56 of the hub 54 and presses down on the hub. The valve flap 55 is movable relative to the boss 50 between the open and closed positions.

The boss 50 also has a valve seat raised rim 65 which extends around the boss 50 to lift the outer periphery of the valve flap 55. This assists in preventing adhesion between the valve flap 55 and the boss 50, facilitates opening of the valve even if the inhalation force applied is low, and ensures sufficient preload to maintain a seal.

The housing 2 also has an oxygen supply port 58 for connection to a supply of supplemental oxygen. In this case the oxygen supply port 58 is located in the base 7 of the device within the margins of the boss 50 and it is normally closed by the inlet valve flap 55. Thus, the inlet valve flap 55 occludes the oxygen port when no oxygen flow is connected, thus maximising device efficiency. When an oxygen supply is connected to the port 58 the valve flap 55 opens. This arrangement avoids the necessity for a separates cap or valve on the oxygen supply port and diffuses oxygen flow entering the chamber 5 which improves the efficiency of the device.

A longitudinal axis through a centre of the aerosol delivery port 6 is substantially at right angles with respect to a longitudinal axis through the housing 2. This assists in reducing aerosol impaction and therefore maximises dose efficiency during changes in flow direction associated with inhalation and exhalation. It also facilitates nebuliser placement for cable management and device usability.

A longitudinal axis through a centre of the patient port 8 is offset from a longitudinal axis through a centre of the valve 53. This feature also assists in reducing aerosol impaction and therefore maximises dose efficiency. This optimum placement also minimises device size and therefore maximises usability.

The main body of the housing 2 comprises a tapered transition section 9 to the patient port 8. This provides minimum resistance to flow and minimises rain-out whilst maximising efficiency.

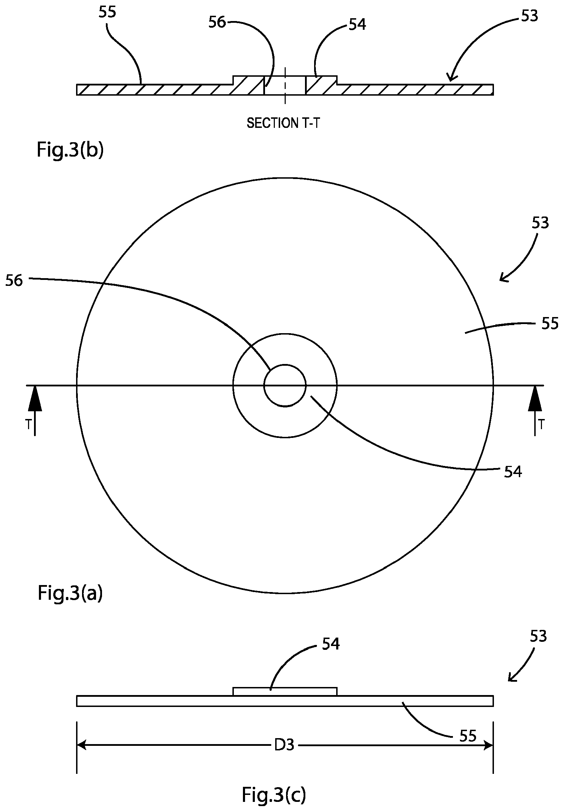

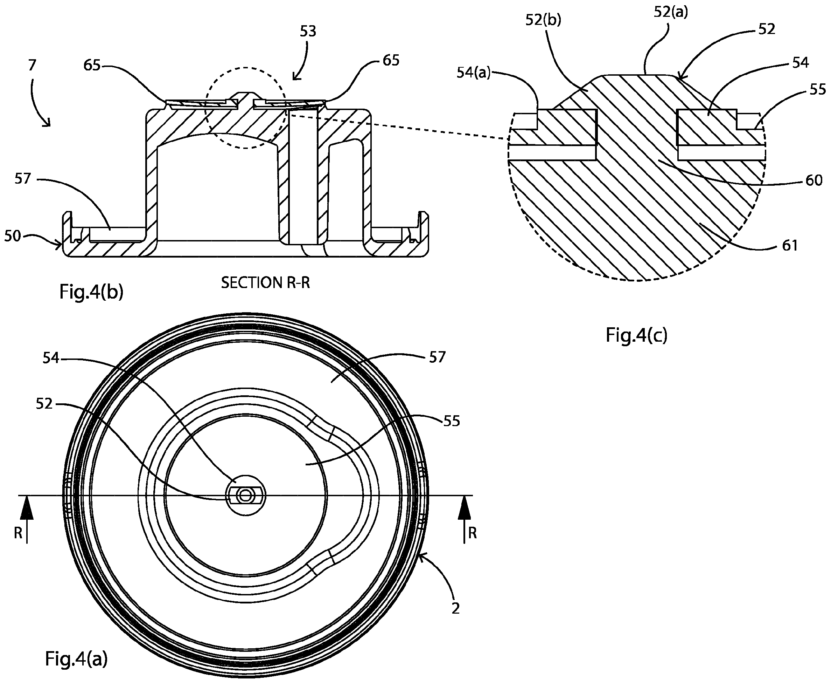

The valve flap 54/55 is shown most clearly in Figs. 3 to 5. It comprises the central annular hub 54 and the surrounding annular flap 55 of smaller thickness. As shown in Figs. 4(a) to (c) the hub 54 is retained in place by the retainer 52 which has a central on-axis body 52(a) and tapered outer ends 52(b) which narrow radially towards its periphery. The tapered ends 52(b) terminate before the edge 54(a) of the hub 54. As shown in Fig. 4(a) the retainer 52 is elongate as viewed in plan, however in other embodiments it extends all of the way around to have a circular outer edge.

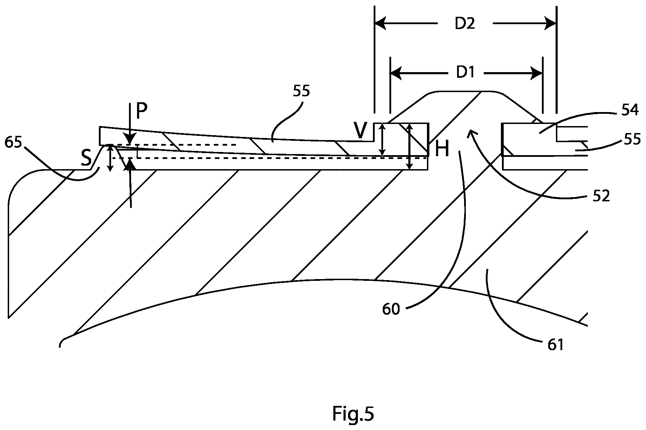

As shown in Fig. 5 the maximum diameter of the retainer 52, where is presses against the hub 54, is D1, and the diameter of the hub 54 is D2. In one preferred embodiment D1 is about 85% of D2. Also, as shown in Fig. 5 the depth (thickness, axial dimension) of the hub 54 is denoted V and is less than a distance H between the retainer 52 and a base 61 of the valve seat 60. This provides on-axis freedom of movement of the valve hub 55 to an extent of H-V. This diagram also shows the extent of axial movement S allowed for the outer edge of the flap 55 in its normally closed position. This diagram also shows the extent of the axial pre-load (P) of the outer edge of the flap 55.

The valve pre-load displacement P (units in m) is determined as follows:

$$P = S - H + V$$

where

S is the height of the sealing ring 65,
H is the axial dimension (height) between the valve seat 60 and the retainer 52, and
V is the thickness of the valve hub 54.

The valve pre-load distance P is preferably in the range of 18% and 157% of the thickness of the valve annular flap 55, more preferably 48% to 128%. Also, the valve pre-load distance P is preferably in the range of 0.5% to 3.5% of the diameter of the valve flap 55.

The device 1 addresses the technical problem of achieving more silent operation, especially where noise would be uncomfortable for consumers, such as in medical settings. The raised rim 65 has the effect of placing the valve flap 55 in a state of flex or pre-load, and this helps to ensure adequate sealing. However, this negatively impacts operating pressures, making the device more difficult to inhale through and can introduce acoustic noise.

The silicone valve flap 54/55 is held by the clip or retainer 52 in a manner where the valve hub 54 is not held in compression at the centre and the preload of the valve is reduced to ensure full sealing but also that it can open easily on activation of flow through it. Also, there is less risk of the valve hub 54 and flap 55 being under excessive stress and thereby stiffening.

It was found that because D1 is less than D2 by at least 10% (preferably in the range of 80% to 90%, and more preferably 83% to 87%) the shorter retainer (or "clip") overhang reduces the tendency of the valve flap 55 to flutter and cause noise. These benefits are achieved primarily because (a) the maximum radial dimension D1 of the clip 52 is less than the radial dimension D2 of the hub 54, and because there is axial freedom of movement of the hub to the extent of the difference between the axial dimensions V and H. In the example illustrated the clip 53 is elongate (see plan view of Fig. 4(a), and D1 is the maximum radial dimension of the clip. The clip has a smaller radial dimension across its width (vertical direction as viewed in Fig. 4(a). However, the invention also achieves a reduction in acoustic noise and valve flutter if the clip is round in plan view, having the same radial dimension in all radial directions. The smaller radial dimension of the clip gives additional freedom of movement to the hub 54 near the hub edge 54(a), contributing to a lower valve stiffness and greater movement is allowed for the flap 55.

Valve stiffness is related to the natural frequency of the valve. Higher valve stiffness for example relates to a higher natural frequency of the valve. Tuning of the natural frequency can help to reduce acoustic noise and valve flutter. Sufficient stiffness must remain in the valve such that the valve remains seated on the raised ring 65 in all orientations, including the orientation where the device is fully inverted such that gravity directly acts to try and open the valve.

Valve stiffness is also related to the valve opening pressure and operating pressure. If the valve is too stiff it will be difficult to open and is more likely to encounter a valve flutter situation. On the other hand, where the valve is soft, the opening pressure and operating pressure will be low which is beneficial for breathing apparatus, however, if the valve is not stiff enough, again it will not create a sufficient seal so a balance must be struck.

Valve stiffness can be controlled by various factors. Examples of factors that increase valve stiffness are a smaller diameter D3 of the valve flap 54/55 (Fig. 3(c)), smaller radial distance between the valve axis and the raised rim 65, taller raised rim 65, valve hub 54 held in axial compression by the valve clip 52, stiffer valve material, larger clip 52 width D1, larger thickness hub 54 and valve 55. The opposite of these statements would serve to lower valve stiffness.

A major benefit of the invention is that it greatly reduces or eliminates valve flutter, which can cause audible valve hum. Valve hum is caused by air flowing past the valve at a pressure greater than the valve opening pressure then, once the valve opens, the flowing air drops in pressure to that less than the valve opening pressure causing the valve to close once again. As the pressure builds again, the valve opens once again and so on such that it opens and closes at a particular frequency. In many situations it is at a frequency that is audible to humans.

For breathing applications, a soft open valve is preferred to prevent putting unnecessary pressure on the lungs of ill patients. The invention satisfies this with a very low opening pressure and flow pressure needed to maintain the open position of the valve.

To reduce the potential for valve hum, whilst ensuring full sealing of the valve a balance must be sought between enough sealing (contact) pressure at the valve flap (55) to seat (60, 65) interface yet not too much that it is difficult to open the valve.

The valve 53 ensures full sealing, without a leak path at low pressures. There is no valve flutter or audible valve hum during operation of the valve at flow rates up to 150 litres per minute. There is a low operating pressure to keep the valve open during use (> -1.5 cm H2O / > -1.47 mbar at an inhalation flow rate of 15 litres per minute), and it remains sealed in all orientations including the upside-down orientation.

Generally, the smaller the retainer radial dimension, the lower the overall stiffness of the valve, the easier the valve is to open and the less likely it is to have valve hum. The only limitation on making it too small is that the valve flap might fall off. It is preferred that the retainer width (D1) should be less than 85% of valve shoulder/hub 54 width (D2).

The valve pre-load P is determined as follows:

$$P_{max} = S_{max} - H_{min} + V_{max.}$$

$$P_{min} = S_{min} - H_{max} + V_{min.}$$

where

S is the height of the sealing ring 65,
H is the axial dimension (height) between the valve seat 60 and the retainer 52,
V is the thickness of the valve hub 54, and
"max" and "min" are the maximum and minimum dimensions allowed by the tolerance windows.

Valve preload directly affects the ease/difficulty of opening the valve and liability to hum. Less preload lends itself to a lower opening pressure, but too little preload can leave the valve sitting in a naturally open position. More preload lends itself to a higher opening pressure which is undesirable for breathing applications, however, it does generally ensure better sealing. In one instance, for a general valve flap thickness of 0.55 mm, it is preferred that valve preload should be between 18% and 157% of the general valve thickness. Also, in this instance the valve preload P (Fig. 5) should be between 0.5% and 3.5% of the valve diameter D3 (see Fig. 3(c).

Tests

An experiment was run using an ASL (Active Servo Lung, Breathing Simulator) device. It used typical healthy adult breathing parameters:

Tidal Volume: 500 mL,
Inspiratory:Expiratory (I:E) Ratio: 1:1, and
Breaths per Minute: 15 BrPM.

The device of the invention used in the tests had the parameters of:

Valve flap 55 diameter, D3 = 20 mm.
Valve hub 54 diameter D2 = 5 mm.
Clip/Retainer 52 maximum dimension D1 = 4.25 mm.
Difference between V and H = 0.28 mm.

In the device of the prior art the clip is pressed against the hub, leaving no axial freedom of movement at the hub, and the clip has the same radial dimension as the hub, pressing down onto the full area of the hub's top surface.

The sound level was recorded using a phone microphone (dB(A) scale) held in controlled location/orientation 100 mm from base of the device. Sound level is left unitless for this reason (non-calibrated equipment). Background noise with just ASL taken as reference.

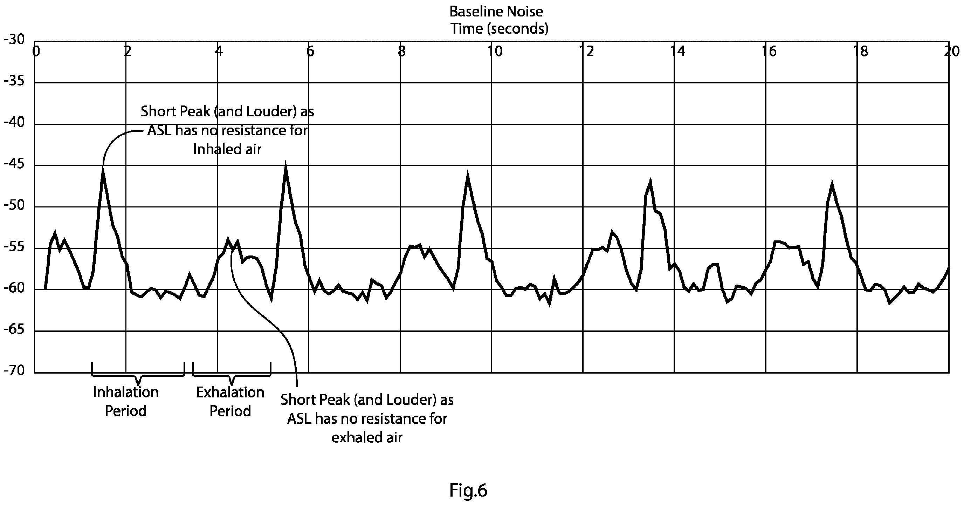

Fig. 6 shows the baseline noise over time. There is slightly higher noise in the inhalation period, but it is generally below -45 dB.

The ASL breathing simulator is for use with various medical devices designed to be used for pulmonary conditions on spontaneous breathers such as nebulisers. The medical devices that connect to the ASL generally provide some form of resistance to the air being inhaled and exhaled by the device during a simulated breath. This has the action of smoothing out the air flow generated by the ASL and consequently the noise. In the background/baseline noise study, the ASL was setup with no connected external equipment hence the inhalation and exhalation were taken sharply and with more noise than if there was connected equipment such as the invention described here.

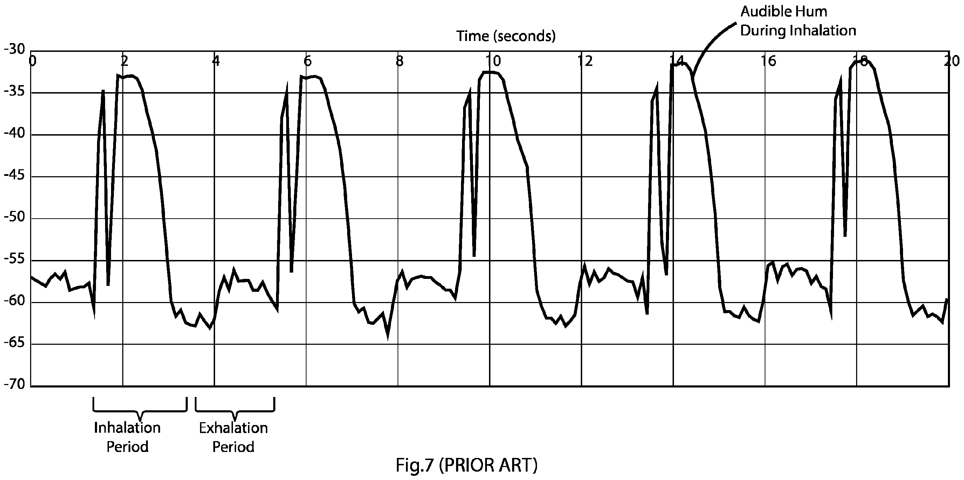

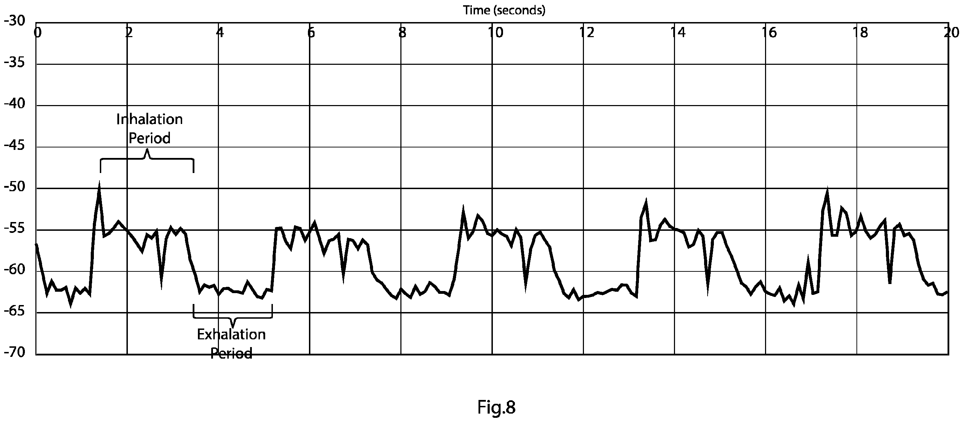

Fig. 7 shows that there is much higher noise, up to -32dB with a device of the prior art, with the retainer extending the full radial dimension of the hub and the hub being pressed against the valve seat. Fig. 8 shows that the noise levels are much lower with a device of the invention.

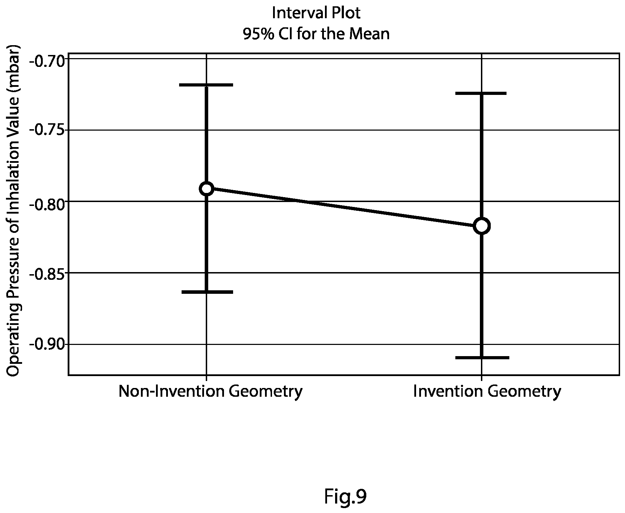

Fig. 9 shows the small nature of the vacuum needed to be drawn in the aerosol chamber during inhalation which is greater than -1 mbar, (closer to 0 mbar is good), to open the inhalation valve and hold it open for the duration of the inhalation. Generally, a valve opening and flow pressure of as low as -1.47 mbar is acceptable. This low inhalation pressure ensures that the valve is not providing too much stress on the lungs of ill patients who may already be struggling to breathe. Maintaining a low opening and flow pressure is very advantageous in the use case of this device and is difficult to achieve whilst keeping the valve in a state where it can remain fully sealed in all orientations. As described above, stiffening of the valve by any means would negatively affect the valve opening and flow pressure. The opposite is also true, in that reducing the stiffness of the valve would serve to reduce the opening and flow pressure of the valve, however, this is limited by the amount of stiffness that must remain in the valve to ensure that it fully seals in all orientations.

Components of embodiments can be employed in other embodiments in a manner as would be understood by a person of ordinary skill in the art. The invention is not limited to the embodiments described but may be varied in construction and detail.

Claims

1. An aerosol delivery device comprising:

a housing (2) defining a chamber,
an air inlet (50) with a one-way valve to allow inflow during inhalation,
an aerosol port (6) for delivery of aerosol into the chamber, and
a patient interface (3) with a one-way valve (22) to allow exhaust outflow during exhalation, wherein:

the inlet valve comprises an annular flap (55) with a central hub (54) through which a retainer (52) extends axially, and the retainer extends radially to hold the hub (54) in place between the retainer and a valve seat (60),
the retainer does not press the hub (54) against the valve seat, a gap (H) between the retainer and the valve seat (60) being greater than the axial dimension (V) of the hub (54).

2. An aerosol delivery device as claimed in claim 1, wherein the retainer (52) has a maximum radial dimension (D1) which is less than a radial dimension

(D2) of the hub (54).

3.  An aerosol delivery device as claimed in claim 2, wherein the retainer maximum radial dimension is less than the radial dimension of the hub by at least 10%.

4.  An aerosol delivery device as claimed in claim 3, wherein the retainer (52) maximum radial dimension (D1) is in the range of 80% to 90% and preferably 83% to 87% of the maximum radial dimension (D2) of the hub (54).

5.  An aerosol delivery device as claimed in any preceding claim, wherein the outer edge of the valve flap (55) is raised out of its resting plane by a pre-load displacement distance (P) by a valve rim (65) at the outer periphery of the valve flap (55) on a valve seat (60), in which:

$$P = S - H + V$$

where,

S is the height of the valve rim (65),
H is the axial dimension between the valve seat (60) and the retainer (52), and
V is the thickness of the valve hub (54).

6.  An aerosol delivery device as claimed in claim 5, wherein the pre-load displacement distance P is in the range of 18% and 157% of the thickness of the valve annular flap (55).

7.  An aerosol delivery device as claimed in claim 6, wherein the pre-load displacement distance P is in the range of 48% to 128% of the thickness of the valve annular flap (55).

8.  An aerosol delivery device as claimed in any of claims 5 to 7, wherein the valve pre-load of the thickness of the valve annular flap (55) distance (P) is in the range of 0.5% to 3.5% of the diameter of the valve flap (55).

Fig.1

Fig.2

55    56    54    53

SECTION T-T

Fig.3(b)

56

53

55

54

Fig.3(a)

54    53

D3    55

Fig.3(c)

53

65                    65

7

57

50

Fig.4(b)          SECTION R-R

52(b)          52(a)          52

54(a)                        54

55

60

61

Fig.4(c)

54

52

55

57

R          R

2

Fig.4(a)

EP 4 670 766 A1

Fig.5

Fig.6

Fig.7 (PRIOR ART)

Fig.8

Fig.9

<table>
<tr><td>Europäisches<br>Patentamt<br>European<br>Patent Office<br>Office européen<br>des brevets</td><td>**EUROPEAN SEARCH REPORT**</td><td>**Application Number**<br><br>EP 24 18 5275</td></tr>
</table>

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A,D | EP 2 868 339 A1 (STAMFORD DEVICES LTD [IE]) 6 May 2015 (2015-05-06) * the whole document * | 1-8 | INV.<br>A61M15/00 |
| A | WO 00/12161 A1 (OTTO SCHILL GMBH & CO KG [DE]; DOETZ KLAUS JOACHIM [DE] ET AL.) 9 March 2000 (2000-03-09) * the whole document * | 1-8 | |
| A | WO 01/34232 A1 (PARI GMBH [DE]; PRE HOLDING INC [US] ET AL.) 17 May 2001 (2001-05-17) * the whole document * | 1-8 | |
| A | WO 02/092146 A2 (CLINICAL TECHNOLOGIES INC [US]) 21 November 2002 (2002-11-21) * the whole document * | 1-8 | |
| A | US 5 617 844 A (KING RUSSELL W [US]) 8 April 1997 (1997-04-08) * the whole document * | 1-8 | |
| A | DE 199 02 847 C1 (KENDALL MED ERZEUGNISSE GMBH [DE]) 18 May 2000 (2000-05-18) * the whole document * | 1-8 | TECHNICAL FIELDS SEARCHED (IPC)<br><br>A61M |
| A | WO 2007/030162 A2 (NEKTAR THERAPEUTICS [US]; CECKA WALTER [US] ET AL.) 15 March 2007 (2007-03-15) * the whole document * | 1-8 | |

The present search report has been drawn up for all claims

| Place of search | Date of completion of the search | Examiner |
|---|---|---|
| Munich | 12 November 2024 | Borowski, Aleksander |

CATEGORY OF CITED DOCUMENTS

X : particularly relevant if taken alone
Y : particularly relevant if combined with another
     document of the same category
A : technological background
O : non-written disclosure
P : intermediate document

T : theory or principle underlying the invention
E : earlier patent document, but published on, or
     after the filing date
D : document cited in the application
L : document cited for other reasons

& : member of the same patent family, corresponding
     document

EPO FORM 1503 03.82 (P04C01)

## ANNEX TO THE EUROPEAN SEARCH REPORT
## ON EUROPEAN PATENT APPLICATION NO.

EP 24 18 5275

This annex lists the patent family members relating to the patent documents cited in the above-mentioned European search report.
The members are as contained in the European Patent Office EDP file on
The European Patent Office is in no way liable for these particulars which are merely given for the purpose of information.

12-11-2024

| Patent document cited in search report | | | Publication date | Patent family member(s) | | Publication date |
|---|---|---|---|---|---|---|
| EP | 2868339 | A1 | 06-05-2015 | NONE | | |
| WO | 0012161 | A1 | 09-03-2000 | AT | E248619 T1 | 15-09-2003 |
| | | | | DE | 19838711 C1 | 21-06-2000 |
| | | | | EP | 1107809 A1 | 20-06-2001 |
| | | | | WO | 0012161 A1 | 09-03-2000 |
| WO | 0134232 | A1 | 17-05-2001 | AT | E404234 T1 | 15-08-2008 |
| | | | | AU | 781911 B2 | 23-06-2005 |
| | | | | AU | 1698301 A | 14-05-2001 |
| | | | | CA | 2389936 A1 | 17-05-2001 |
| | | | | DE | 19953317 C1 | 01-02-2001 |
| | | | | EP | 1227856 A1 | 07-08-2002 |
| | | | | ES | 2311475 T3 | 16-02-2009 |
| | | | | JP | 4589862 B2 | 01-12-2010 |
| | | | | JP | 2003513719 A | 15-04-2003 |
| | | | | JP | 2006122692 A | 18-05-2006 |
| | | | | NZ | 518782 A | 31-10-2003 |
| | | | | WO | 0132246 A1 | 10-05-2001 |
| | | | | WO | 0134232 A1 | 17-05-2001 |
| WO | 02092146 | A2 | 21-11-2002 | AU | 2002305565 A1 | 25-11-2002 |
| | | | | US | 2002121275 A1 | 05-09-2002 |
| | | | | US | 2007169774 A1 | 26-07-2007 |
| | | | | WO | 02092146 A2 | 21-11-2002 |
| US | 5617844 | A | 08-04-1997 | NONE | | |
| DE | 19902847 | C1 | 18-05-2000 | NONE | | |
| WO | 2007030162 | A2 | 15-03-2007 | AU | 2006287934 A1 | 15-03-2007 |
| | | | | BR | PI0610062 A2 | 25-05-2010 |
| | | | | CA | 2607148 A1 | 15-03-2007 |
| | | | | CN | 101184522 A | 21-05-2008 |
| | | | | EP | 1893273 A2 | 05-03-2008 |
| | | | | HK | 1117435 A1 | 16-01-2009 |
| | | | | JP | 5087539 B2 | 05-12-2012 |
| | | | | JP | 2008540056 A | 20-11-2008 |
| | | | | KR | 20080017375 A | 26-02-2008 |
| | | | | US | 2007083677 A1 | 12-04-2007 |
| | | | | US | 2015114504 A1 | 30-04-2015 |
| | | | | WO | 2007030162 A2 | 15-03-2007 |

EPO FORM P0459

For more details about this annex : see Official Journal of the European Patent Office, No. 12/82

**EP 4 670 766 A1**

REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 2868339 A